(19) Europäisches Patentamt
European Patent Office
Office européen des brevets

(11) **EP 4 386 765 A1**

(12) **EUROPEAN PATENT APPLICATION**

(43) Date of publication:
**19.06.2024 Bulletin 2024/25**

(21) Application number: **22214055.0**

(22) Date of filing: **16.12.2022**

(51) International Patent Classification (IPC):
*G16C 20/30* (2019.01)     *G06N 3/00* (2023.01)
*G16C 20/70* (2019.01)

(52) Cooperative Patent Classification (CPC):
**G16C 20/30; G06N 5/01; G06N 20/00; G06N 20/10;
G16C 20/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **BASF SE**
**67056 Ludwigshafen am Rhein (DE)**

(72) Inventors:
• **Gastegger, Michael**
  **10559 Berlin (DE)**
• **Deglmann, Peter**
  **67056 Ludwigshafen am Rhein (DE)**

• **Heilig, Manfred**
  **67346 Speyer (DE)**
• **Schaefer, Ansgar**
  **67056 Ludwigshafen am Rhein (DE)**
• **Hussing, Marcel**
  **64287 Darmstadt (DE)**
• **Pfeifle, Mark Claudius Gerhard**
  **67056 Ludwigshafen am Rhein (DE)**
• **Eiden, Philipp**
  **67056 Ludwigshafen am Rhein (DE)**

(74) Representative: **Eisenführ Speiser**
**Patentanwälte Rechtsanwälte PartGmbB**
**Postfach 10 60 78**
**28060 Bremen (DE)**

(54) **APPARATUS FOR DETERMINING PROPERTIES OF A SUBSTANCE**

(57)     The invention refers to an apparatus 100 for determining properties of a substance. A providing unit 110 provides atomic descriptors that are indicative of characteristics of atoms of the substance with respect to the structure of the specific molecule. A model providing unit 120 provides a trained property model, wherein the trained property model has been trained to determine a property of the substance as output when provided with the atomic descriptors. The property model comprises a) an atomic classifier adapted to classify atoms of the substance into one or more atom classes based on the atomic descriptors and b) a regression model adapted to determine the property based on the atom classes determined for the substance. A determination unit 130 determines the property of the substance by applying the trained property model to the atomic descriptors.

FIG.1

**Description**

FIELD OF THE INVENTION

The invention relates to an apparatus, a method and a computer program product for determining properties of a pure substance or a substance in a given mixture. Further, the invention relates to a training apparatus, a training method and computer program product for training a machine leaning based property model that can be utilized in the apparatus, method and computer program product for determining the aforementioned properties of the substance.

BACKGROUND OF THE INVENTION

Generally, the determination of, for instance, thermodynamic properties of substances defined as being of either molecular nature or representing extended periodic 1D-, 2D- or 3D-structures, is a challenging problem with high industrial relevance. Many established thermodynamic models, in particular in the area of molecular substances, are so called "group contribution" models in which a property is determined out of contributions attributed to hierarchically derived functional groups of a molecule. However, deriving the hierarchy to be followed for decomposing a molecular structure to respective functional groups is not only a cumbersome and lengthy task for an expert or a complicated software, but also is a cause for many inaccuracies in the prediction of the thermodynamic properties. For example, a functional group fitting to the same hierarchical pattern provided in different chemical environment can behave significantly different. However, in current models such differences cannot be taken into account. Thus, such prediction models for thermodynamic properties can often only be applied to a specific and very limited chemical space and thus often do not allow for the industrially relevant screening of huge amounts of molecules for specific properties. Moreover, even to achieve an acceptable accuracy in their limited field of application, these predictive models require a huge amount of training data for training the respective models such that only models can be provided for molecule groups for which such a huge data basis exists already. However, for instance, for quite new functional groups or novel combinations of functional groups in close proximity, there are often not enough data available to achieve an accuracy of such a model that would allow for a sensible application.

It thus would be advantageous if a predictive model for determining physicochemical properties of a substance could be provided that allows for (a) the determination of properties also for substances belonging to other classes than those of the training set and (b) a higher accuracy in determining the property while at the same time requiring less training data.

SUMMARY OF THE INVENTION

It is an object of the present invention to provide an apparatus, a method and a computer program product that allow for a very accurate determination of properties of a substance defined as being of either molecular nature or representing extended periodic 1D-, 2D- or 3D-structures. Moreover, it is further an object of the invention to provide a training apparatus, training method and computer program that allow to provide a predictive model usable in the apparatus, method and computer program for determining properties that (a) is able to determine properties also for substances belonging to other classes than those of the training set and (b) can be trained to provide the high determination accuracy with less training data. The flexibility of the current invention furthermore allows the generation of prediction models for very diverse target properties, because the mathematical structure naturally tailors the model's predictors to the determined property. In contrast to classical group contribution methods, the model does not need to be manually tuned to the target property by an expert. The model also does not require bonds and bond orders between atoms as input, making it applicable to situations where these are hard to establish, e.g. metal complexes, hydrogen bonding.

In a first aspect of the present invention, an apparatus for determining properties of a substance defined as being of either molecular nature or representing extended periodic 1D-, 2D- or 3D-structures is presented, wherein the apparatus comprises i) an atomic descriptors providing unit for providing atomic descriptors that are indicative of characteristics of atoms of the substance with respect to the structure of the specific molecule, ii) a trained property model providing unit for providing a trained machine learning based property model, wherein the trained property model has been trained to determine a property of the substance as output when provided as input with the atomic descriptors, wherein the property model comprises a) an atomic classifier adapted to classify atoms of the substance into one or more atom classes based on the atomic descriptors and b) a regression model adapted to determine the property based on the atom classes determined for the substance, iii) a property determination unit for determining the property of the substance by applying the trained property model to the atomic descriptors and iv) a property providing unit for providing the property for further processing.

Since the property model comprises a) an atomic classifier adapted to classify atoms of the substance into one or more atom classes based on the atomic descriptors and b) a regression model adapted to determine the property based on the atom classes determined for the substance, in particular, since the atomic classifier and the regression model are both part of a machine-learning based property model and thus can be trained concurrently, atomic classes can be utilized that are specifically determined for the respective training situation.

Thus, the atomic classes utilized for the determination of the property are not dependent on respective insights into the physical or chemical nature of the molecules provided by an expert or used as scientific convention, but are themselves determined by the training specifically for the respective training situation, where the classification can differ from one considered property to another. In addition, these atomic classes map complex structural and chemical motifs into a lower dimensional space composed of a limited number of categories. This allows to train the property model utilizing less training data and further allows for a higher accuracy in determining the property for the respectively trained situations and finally also allows to make predictions for substances containing functional groups not covered by the training set. All this is a consequence of the fact that the thus performed classification is more targeted towards atomic features that really matter for the property of interest.

Moreover, due to the incredibly high number of possible, often not even fully explored substances, potentially suitable for a specific application, today a technical product engineer, given the technical task of finding a substance that is not only suitable for a specific application, but also fulfills respective numerous further target properties has to synthesize and test huge amounts of possible substances, or go through huge datasets and libraries in which potential substances are stored in order to find a respective substance that might fit the application. Even when utilizing sophisticated design of experiment methods, still a very high number of possible substances has to be synthesized and experimentally tested. In this context the above described method allows to assist a user, for instance, a technical product engineer, to find potentially suitable substances automatically and much faster. In particular, by utilizing the above method the user only has to synthesize and test much less potentially suitable substances for which it has been determined that it is very likely that they fulfill the respective target property. Accordingly, unnecessary synthesizing and testing of substances can be avoided. Thus, the method allows a user to perform a technical task of finding a substance suitable for a technical application faster and more efficiently.

Generally, the substance, for which a property should be determined, is considered as being of either molecular nature or representing extended periodic 1D-, 2D- or 3D-structures. Preferably, the substance is of a molecular nature, i.e. is a molecular substance, even more preferably, the substance is of a molecular nature and contains at least one of the following elements H, C, N, O, F, Si, P, Cl, Br and I. Generally, the principles of the present invention can be applied to any type of substances for which a respective training data set is present. However, the larger the intended application range the model should be applied to, the more training data has to be provided for reaching a sufficient accuracy. Furthermore, it is preferred that molecular substances have a molecular weight of less than 600 g/mol, more preferably of less than 300 g/mol. Further, it is preferred that the specific molecule is present in the environment in a form that allows to completely describe the molecule using simple structural formulas, that contain the relevant information. A simple molecular structure refers to molecules that can be unambiguously described by covalent bonds between the atoms of the molecule. Examples, where this is not the case, are e.g. systems with dynamic equilibria between several forms like monomer and oligomers as in the case of several inorganic acids, or ionic species with very localized charge that strongly interacts with a solvent, e.g. via hydrogen bonding. The substance can generally refer also to a periodic system, i.e., a substance in which the specific molecule is repeated structurally in a defined manner in space, for instance, in such cases a substance can refer to a crystalline metal or semiconductor or other systems comprising a periodic atomic symmetry.

The atomic descriptors providing unit is adapted to provide atomic descriptors. In particular, the atomic descriptors providing unit can be a storage unit or can be in communicative contact with a storage unit on which the atomic descriptors are already stored. However, the atomic descriptors providing unit can also refer or be in communicative contact with an atomic descriptors determination unit for determining the atomic descriptors, for instance, based on structural information of molecule substance. Preferably, the atomic descriptors are indicative of characteristics of atoms of the substance with respect to an atomically resolved 3D-structure. Preferably, the atomic descriptors comprise atom specific quantities derived from the atomic structural environment of the atoms of a substance and from its electronic structure of the molecule.

The derivation of atomic descriptors from an atomic structural environment can be performed according to any formalism which can transform a three-dimensional structure and elemental composition of a molecule system to a set of atom-wise descriptors. These can be end-to-end architectures like SchNet, PhysNet but also pre-defined atomic descriptors, e.g. ANI, SOAP, FCHL, can be used. Atomic descriptors derived from molecular electronic structure calculations can refer, for instance, to one or more of partial charge, exposed surface fraction, averaged surface screening charge density from continuum solvation model calculations, averaged atomic radius in continuum solvation model calculations based on using a flexible, e.g. iso-density cavity, contributions to energy or free energy in COSMO-RS- or COSMO-SAC-like solvation models, e.g. misfit-, H-bond-donating, H-bond-accepting, dispersive or total residual contribution, pairwise contact probabilities from COSMO-RS- or COSMO-SAC-like solvation models, NMR-shielding constant.

The trained property model providing unit is adapted to provide a trained machine learning based property model. In particular, the trained property model is adapted, i.e. has been trained, to determine a property of the substance as output when provided as input with the atomic descriptors. The determined property can refer to any property of a substance for which the trained machine learning based property model has been trained. In particular, in preferred embodiments, the property is a physicochemical property that can refer to any of a melting point, a boiling point, a vapor pressure, a heat of vaporization, a heat capacity, a flashpoint, an auto ignition temperature, a liquid density, a critical temperature and/or pressure, an electric and/or a thermal conductivity, a glass transition temperature, a viscosity, a surface tension, a refractive index, a general mechanical property, a total energy, a dipole moment, a polarizability, HOMO-LUMO- or bandgap, an ionization potential, an electron affinity, an activity coefficient, a partition coefficient, a solubility, a cloud point, a critical micelle concentration, an acid and base dissociation constant, hydrolytic stability, a corrosivity, and a catalytic activity for a given chemical reaction, or a spectroscopic property, e.g., transition energies and intensities. Further, in preferred embodiments, the application properties can refer to any of a) general properties beyond pure physical chemistry, e.g. various types of toxic and eco-toxic behavior, biodegradability in various habitats, odor, ozone depletion potential, global warming potential, etc., and b) performance properties as an additive in mixtures e.g., influence on octane and cetane number of fuels, stabilization against UV-radiation or oxidation, anti-corrosive action on surfaces in solutions or coatings, flame retardancy, influence on haptics, etc.

In a preferred embodiment, the determined property refers to a melting point of the substance. Determining the melting point of a substance utilizing the property model allows to determine very fast, i.e. without having to synthesize the substance and perform a complex measurement sequence, whether a substance in question is suitable for agrochemical or pharmaceutical application, or, for instance, as resin raw material, e.g., for epoxy- or polyurethane systems. In particular, for these applications, it is important that a substance in question does not possess a too high melting point. Thus, utilizing the property model potential substances can be scanned very fast for their general suitability. Additionally or alternatively, in an embodiment, the property refers to vapor pressure. For example, if the substance refers to a potential plastic adaptive, in this way it can be checked before the synthesis of the plastic additive or the preparation of samples for application testing, if the volatility of the substance is low enough to ensure that the additive substance stays within the desired material maintaining its effects. This can, for instance, increase the security of the synthesis product. Moreover, the determination of vapor pressures for a substance allows together with other properties like activity coefficients, for instance, for the identification of suitable conditions for separation processes, e.g. distillation. Additional or alternatively, in an embodiment, the property refers to a thermal conductivity. This allows the application of the apparatus to the search for polymer materials that can act as thermal conductors or insulators and allows for virtual screening for new insulating materials that can be used, for instance, for selecting appropriate materials for electric devices that avoid an overheating. Additional or alternatively, in an embodiment, the property refers to the viscosity. Utilizing the apparatus to determine the viscosity of a potential substance allows to determine if the viscosity is in a given range, for instance, in the context of polymer processing, e.g. utilizing reactive resins, or extrusion processes followed by injection molding or film blowing. Additional or alternatively, in an embodiment, the property refers to a solubility. Screening potential substances using the apparatus with respect to solubility can be advantageous in process designing for finding, for instance, an ideal solvent and/or precipitant for a given chemical synthesis or extraction/purification workout. Moreover, without having to utilize complex testing sequences, it can be ensured that a potential active ingredient, i.e., substance, is available for a biological uptake or that no demixing takes place in formulated products. Additional or alternatively, the property refers to an ionization potential. Training the property model for determining an ionization potential allows to apply the apparatus for the screening of potential battery materials, e.g., matrices, solvents, membranes that are resistant to oxidation. Moreover, the ionization potential can also be utilized as a physical descriptor for a molecule that can correlate with other application properties such that the determination of an ionization potential can itself again be utilized in the context of another trained property model determining another property of a substance. Additionally or alternatively, in an embodiment, the property refers to one or more spectroscopic properties, for instance, transition energies and/or intensities. In this case, the apparatus can also be applied in verification processes, in which it has to be verified that a synthesis step has indeed yielded the desired product. Additional or alternatively, the property refers to a flame retardancy, either of the substance itself as a material or of a physical mixture of a polymer and the substance which then serves as flame retarding additive. Determining a flame retardancy for additivated polymers allows for a virtual screening to find new flame retardants that are more efficient than existing substances in plastic formulations. Moreover, also inherently flame retardant materials can be searched very fast and effectively in this way.

The trained property model comprises a) an atomic classifier adapted to classify atoms of the molecule into one or more atom classes based on the atomic descriptors and b) a regression model adapted to determine the property based on the atom classes determined for the substance. In particular, the atomic classifier can refer to any machine learning classifier that can be trained to provide one or more classes for objects and to classify the object into the respective classes based on the provided characteristics of the objects, in this case, based on the provided atomic descriptors. In particular, the atom classes utilized by the atomic classifier are not predetermined, for instance, by user considerations or an expert class hierarchy, but are trained concurrently with the regression model such that for each respective case, for instance, for each respective property that should be determined, the optimal atom classes are identified by training alone. Thus, the atom classes of the atomic classifier are determined in a purely data-driven manner. Also, the regression model that utilizes the atom classes determined for the molecule to determine the property can refer to any machine learning based regression model. Generally, a machine learning regression model refers to any machine learning model that assigns a continuous dependent variable to a set of input variables, wherein in the present application the continuous dependent variable refers to the property and the set of input variables to the values of the respective atom classes. Preferably, the regression model refers to anyone of a multilinear regression, an artificial neural network, Gaussian process regression, a Kernel algorithm, a support vector regression and a random forest algorithm. Utilizing a classifier that can learn the atom classes based on the training data and a following regression model has many advantages. In particular, the atom classes in this case provide a description of the characteristics and influences of the respective atoms of the substance on a property that is learned from the training data and thus is specific for each application and independent of already known convenient classifications. This allows to provide intrinsic information already as part of the model leading to the possibility of reducing the amount of training data, for instance, by reducing the dimension of the information that is to be provided by the training data. Moreover, it becomes possible to train the model to utilize more meaningful descriptors that allow also to interpret the results of the training, i.e. the property model. Further, the inventors have found that the above property model is more robust with respect to artefacts that can be caused by biased training data.

The property determination unit is then adapted to determine the property of a substance by applying the trained property model to the atomic descriptors. In particular, the atomic descriptors are provided as input to the trained property model, which then provides the determined property for the substance as output. The determined property can then, for instance, by a property providing unit be provided for further processing. For example, the further processing can refer to providing the determined property as output to a user, for instance, via a screen or any other suitable way. However, the determined property can also be provided for further processing with respect to specific applications, for instance, can internally be used in a screening process, screening a plurality of substances, wherein in this case the respective property is not necessarily provided to a user, but, for instance, only used for determining a list of potential suitable substances for an application and further processed, for example, by an automatic synthesis unit for automatically synthesizing the substances on the list . Moreover, the determined property can also by further processed by being provided to a database and then stored before being further processed, for instance, in a search operation of the database.

Preferably, the processing of the determined property comprises determining control signals for controlling a production process based on the determined property. The production process can refer to a production process of the substance or can refer to a production process of a product in which the substance is utilized. For example, if the determined property indicates that substance possesses a specific vapor pressure the generation of the controlling signals can comprise generating controlling signals for controlling a production process of a polymer utilizing the substance and taking the vapor pressure of the substance into account. In a preferred embodiment the control signal is indicative of a machine executable formulation of the substance, in particular, when a comparison indicates that the determined property of the substance lies within a predetermined range around a provided target property.

Moreover, the process of processing the property can also refer to a step of selecting one or more substances based on respectively determined properties. For example, if for a plurality of potential substances respective properties have been determined, the selecting can comprise comparing the determined properties of the different substances to predetermined selection criteria and select the substances for which the determined properties fulfill these criteria. In particular, in an embodiment the method comprises receiving a target property for a substance and comparing the received target property with a determined property and providing depending on the comparison a control signal. The control signal can refer to any signal that allows for a further control of a technical system. For example, the control signal can be adapted to control an interface for providing the result of the comparison on the interface. In a preferred embodiment the comparison refers to a validation of the target property, wherein the validation is positive if the determined property falls within a predetermined range around the target property. In this case the control signal can be adapted to simply control a user interface to provide an indication of a positive or negative validation result. However, preferably, the control signal refers to a recipe, i.e. a formulation, of the one or more substances which fulfill the specified target property, i.e. which are validated positively. A recipe is generally defined as an instruction on how a substance can be synthesized or formulated. In particular, the recipe comprises the starting substances and the respective parameters for generating the substance from the starting substances. Preferably, the control signals comprise a recipe in a form that directly allows an automatic controlling of respective industrial systems or labor equipment for producing the substance. In particular, it is preferred that the control signal is indicative of a machine executable recipe of the substance, when the result of the comparison refers to the determined property being within a predetermined range around the target property.

In an embodiment, molecular substances are taken into account in more details as an ensemble of several conformers. In this case, the atomic descriptors of the molecule can refer to a weighted average of the atomic descriptors of the different conformers of the substance. Generally, a conformation refers to the phenomenon of conformational isomerism in which isomers can be interconverted by rotations around chemical bonds, mainly formal single bonds, but also by other types of interconversion like ring-flips in cyclic non-aromatic hydrocarbons or pseudorotations. For all of these potential interconversions the criterion holds that they are kinetically possible, i.e. actually occur at a reasonable timescale, at the temperature of interest. In this case, the atoms can be present in the substance in different environments that can lead to different atomic descriptors for different conformers. Here, the accuracy of the determination can be increased by utilizing atomic descriptors referring to a weighted average of the atomic descriptors of the different conformers. In particular, the weights can be indicative of the probability that a specific conformer of the molecule is present in the substance. For example, if it is expected that two possible conformers have the same probability to be present in the substance, the weights can be indicative for this ratio of conformers as expected in the substance. Preferably the weights of the weighted averaging refer to Boltzmann weights. The required conformational energies or free energies for determining the Boltzmann weights can be obtained from calculations based on quantum chemical, semiempirical or machine learning calculation, Moreover the weights can also refer to trained weights trained together with the property model based on the same training data used for training the property model.

In an embodiment the atomic classifier is provided with predetermined constraints for the classification of the atoms of the substance based on the atomic descriptors. Providing predetermined constraints to the classification allows to further increase the efficiency of the classification by constraining the classification, for instance, to chemically sensible classes. Preferably, in an embodiment, the atomic classifier is adapted to classify the atoms of the substance into the one or more atom classes element specific such that atoms referring to different elements are classified into different atom classes. Providing this constraint to a training of the atomic classifier, allows to directly take into account that in many cases, different elements will have different chemical characteristics that have different influences on the property. Thus, this constraint can allow to further reduce the amount of training data necessary for training the property model. However, if a more flexible property model is desired, the atomic classifier can also be trained without any element specific restrictions to the atom classes that are built up by the atomic classifier during the training.

In an embodiment, the atomic classifier is adapted to classify an atom of the substance into one or more atom classes, wherein, if the atom is classified into more than one atom class, an atomic class weight is provided to each atom class to which the atom has been partially assigned based on the fit of the given atom properties to the class definition. In some cases, atoms might comprise characteristics, i.e. atomic descriptors, that lead to an assignment of the atom to more than one atom class. In this case, the atom can then be regarded as influencing the respective property in accordance with both atom classes. However, since in this case only one atom has the effect associated with the respective atom classes, the atomic class weights are provided to prevent that this one atom potentially has the same effect on the properties as a molecule comprising one atom in each of the respective atom classes. In particular, the atomic class weights are assigned based on the the fit of the given atom properties to the class definition with respect to the respective property. However, in an embodiment, the classifier can also be adapted to allow only for a classification of an atom in one atom class. This leads to less flexibility in the training process, but allows for a further reduction of the training data.

In a further aspect of the present invention, a training apparatus for training a machine learning based property model is presented, wherein the apparatus comprises i) a training data providing unit for providing training data for training the property model, wherein the training data comprises atomic descriptors and a corresponding known property of a plurality of different substances, respectively, ii) a trainable property model providing unit for providing a machine learning based property model, wherein the property model comprises a) an atomic classifier trainable to classify atoms of a substance into one or more atom classes based on atomic descriptors, and b) a regression model trainable to determine the property based on the atom classes determined for the molecule, and iii) a training unit for training the provided property model based on the training data such that the trained property model is adapted to determine a property of a substance as output when provided as an input with atomic descriptors of the substance.

Generally, the training data comprising atomic descriptors and correspondingly known properties of a plurality of different substances can be provided in form of a database in which the characteristics and properties or substances are stored. Such databases can be based, for instance, on simulation data, on measurement data, on sequencing data, etc. Preferably, the training data only refers to one property of a substance such that the property model trained based on this training data is dedicated for determining the respective property. However, also more than one property can be provided in the training data such that the training property model can then determine more than one property for each substance. Generally, any know training algorithms for training data-driven, in particular, machine learning based models can be utilized. Preferably, during the training of the property model also the descriptors of the substance that have the most influence on the property are determined and the model is then trained based on these most influential descriptors. For determining these most influential descriptors, for example, cluster analysis or PCA analysis tools can be utilized. In particular, the descriptors can be utilized to determine the application space of the training data, wherein the application space is then defined by the descriptors of the substance that are covered by the data. The determination of the most influential descriptors can then be performed as a dimension reduction of the application space. Then algorithms for optimizing the training data in the application space can be applied, for instance, to cover the application space with as few training data as possible.

Preferably, the training of the property model comprises iteratively optimizing model parameters of the provided property model based on the training data until a predetermined accuracy improvement is provided by the trained property model. In particular, the model parameters comprise classification parameters and regression parameters and the iterative optimization of the model parameters comprises an optimization of the classification parameters of the atomic parameters and of the regression parameters of the regression model with each iteration step. Generally, the accuracy improvement can refer, for instance, as typical for iterative approaches, to a difference between a result of a current iteration step and a previous iteration step. When the iteration converges, as should normally be the case, the difference between the results of each iterative step becomes smaller with each step, wherein the iteration can be stopped if this difference, i.e., the accuracy improvement, lies below a predetermined accuracy improvement, i.e., a predetermined threshold. Possible variations of this procedure comprise to maintain certain parameters over several iterative steps before adapting them again or to apply convergence criteria that take into account the development of accuracy over several iterative steps.

In a further aspect a system is presented for screening for a potential substance in a pre-defined application, wherein the system comprises a) an apparatus as described above, b) a target property providing unit for providing a target property of a target substance and a potential target substance, and c) a screening unit, wherein the screening unit is adapted to utilize the apparatus to determine a respective property of the potential target substances and to compare the determined property of the potential target substance with the target property and, based on the comparison, either i) determining the potential target substance as the target substance, or ii) providing a new potential target substance and repeating the determination of the property utilizing the new potential target substance.

The target property providing unit is configured for providing a target property that is indicative of a characteristic of a substance. In particular, the providing can refer to receiving the target property from an input of a user using, for instance, a respective input unit. Moreover, the providing can also refer to accessing a storage unit on which a target property is already stored. Further, the providing can also comprise receiving a target property, for instance, via a network connection from other sources and providing the received property. Generally, the target property can refer to one target value, for instance, a target vapour pressure, or can refer to a value range that should be met by the substance. Moreover, the target property can also refer to any kind of target function, for instance, a timely sequence of the property. Further the target property providing unit is configured for providing a potential target substance. In particular, the providing can referto receiving the potential target substance from an input of a user using, for instance, a respective input unit. Moreover, the providing can also refer to accessing a storage unit on which the potential target substance is already stored. Further, the providing can also comprise receiving potential target substance, for instance, via a network connection from other sources and providing the received potential target substance. The potential target substance can then be regarded as a starting substance for screening for a substance meeting the target property. The respective descriptors of the substance can be determined based on the provided potential target substance, for example, as already described above. Generally, the descriptors can be determined by accessing a storage unit on which for respective substances the descriptors are already stored. Also any above described method for determining the descriptors based on a provided substance can be utilized.

After utilizing the property model for determining a property for the potential target substance, the determined property is compared with the target property. Based on the comparison it is decided if the potential target substance is determined as the target substance, wherein in this case the iteration can stop at this point. Moreover, based on the comparison it can also be determined to provide a new potential target substance and to repeat the determination of the property utilizing the new potential target substance. Thus, at this point an iteration is performed in which the determination of the property using the property model and the descriptors of a potential target substance is repeated until one of the potential target substances is determined as the target substance. In particular, the comparison can comprise determining whether the determined property of a potential target substance lies within a predetermined range around the target property, wherein in this case the target can be regarded as being fulfilled and the potential target substance is determined as target substance. If the determined property lies outside of the predetermined range around the target property, it is determined that the target is not fulfilled and a new potential target substance is provided that might fulfil the target property.

Generally, the performed iteration can refer to an arbitrary search of the potential target substance space or to a directed search. For example, a new potential target substance can simply be selected arbitrarily from a huge amount of *in-silico* generated potential target substances. However, also specific rules for generating a new potential target substance can be applied based on the comparison between the determined property and the target property, with or without considering the simultaneous optimization of additional target properties of the substance. Generally, known methods for generating new target substances can be utilized, for example, evolutional algorithms or Bayesian optimizers can be used. For example, such algorithms can be used to vary molecular or periodic atomic structures of the substance to generate a new potential target substance.

The iteration can then be performed over the steps of determining the property of the new potential target substance by utilizing the descriptors of the new potential target substance as described above. Optionally, also a determination of descriptors from the potential target substance can be part of the iteration, if the descriptors are not already provided or present on a storage unit. Moreover, it is preferred that the same property model is used in all iteration steps for determining the property. However, in some cases also different properties models can be used in different iteration steps. For example, if other descriptors for the new potential target substance are utilized also another property model can be more suitable. This can be, for example, the case if some descriptors are not available or not available with a suitable accuracy for a new potential target substance.

After the iteration has stopped, for instance, after the potential target substance has been determined as the target substance, or if no new potential target substance can be selected or generated, the result of the iteration can be provided to a user. For example, if none of the possible potential target substance has met the target property, the user can be notified of the failure of determining a target substance. In case a target substance can be determined, the target substance can be provided to the user as output. For example, the determined target substance can then be provided to an output unit or to a computing unit for further processing. Preferably, the providing of the target substance leads to a further processing generating control signals, for example, as already described above.

In a further aspect of the invention a method for determining properties of a substance defined as being of either molecular nature or representing extended periodic 1D-, 2D- or 3D-structures is presented, wherein the method comprises i) providing atomic descriptors that are indicative of characteristics of atoms of the substance with respect to the structure of the substance, ii) providing a trained machine learning based property model, wherein the trained property model is adapted to determine a property of the substance as output when provided as input with the atomic descriptors, wherein the property model comprises a) an atomic classifier adapted to classify atoms of the molecule into one or more atom classes based on the atomic descriptors and b) a regression model adapted to determine the property based on the atom classes determined for the substance, and iii) determining the property of the substance by applying the trained property model to the atomic descriptors.

In a further aspect of the invention, a training method for training a machine learning based property model is presented, wherein the method comprises i) providing training data for training the property model, wherein the training data comprises atomic descriptors and a corresponding known property of a plurality of different substances, respectively, ii) providing a machine learning based property model, wherein the property model comprises a) an atomic classifier trainable to classify atoms of a substance into one or more atom classes based on atomic descriptors, and b) a regression model trainable to determine the property based on the atom classes determined for the molecule, and iii) training the provided property model based on the training data such that the trained property model is adapted to determine a property of a substance defined as being of either molecular nature or representing extended periodic 1D-, 2D- or 3D-structures as output when provided as an input with atomic descriptors of the specific molecule.

In a further aspect, a method is presented for screening for a potential substance in a pre-defined application, wherein the method comprises a) providing a target property of a target substance and a potential target substance, and b) utilizing a method as described above to determine a respective property of the potential target substances and to compare the determined property of the potential target substance with the target property and, based on the comparison, either i) determining the potential target substance as the target substance, or ii) providing a new potential target substance and repeating the determination of the property utilizing the new potential target substance. In a further aspect of the invention, a computer program product for training a machine leaning based property model is presented, wherein the computer program product comprises program code means for causing the property model training apparatus as described above to execute the training method as described above.

In a further aspect of the invention, a computer program product for determining properties of a substance is presented, wherein the computer program product comprises program code means for causing the apparatus as described above to execute the method as described above.

It shall be understood that the apparatuses as described above, the methods as described above and the computer program products as described above have similar and/or identical preferred embodiments, in particular, as defined in the respective dependent claims.

It shall be understood that a preferred embodiment of the present invention can also be any combination of the dependent claims or above embodiments with a respective independent claim.

These and other aspects of the present invention will be apparent from and elucidated with reference to the embodiments described hereafter.

BRIEF DESCRIPTION OF THE DRAWINGS

In the following drawings:

Fig. 1 shows schematically and exemplarily an embodiment of an apparatus for determining properties of a substance,

Fig. 2 shows schematically and exemplarily an embodiment of a method for determining properties of a substance,

Fig. 3 shows schematically and exemplarily an embodiment of an apparatus for training a property model,

Fig. 4 shows schematically and exemplarily a flow chart of a method for training a property model,

Fig. 5 and 6 show schematically an exemplary application of the invention,

Fig. 7 shows schematically and exemplarily an application of the invention for optimizing a formulation, and

Fig. 8 shows schematically and exemplarily a method for increasing an accuracy of the determination of a respective property model and/or for screening for new substances for an application.

DETAILED DESCRIPTION OF EMBODIMENTS

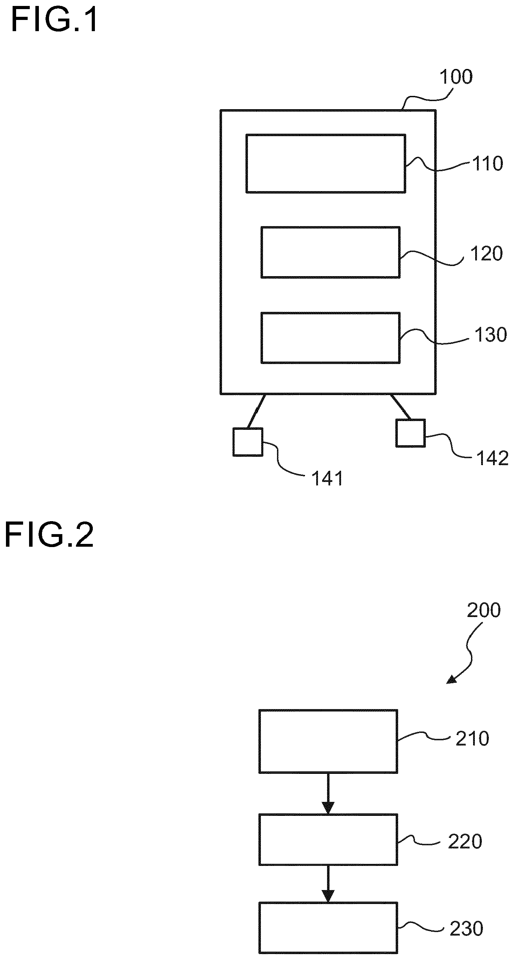

Fig. 1 shows schematically and exemplarily an embodiment of an apparatus 100 for determining properties of a substance defined as being of either molecular nature or representing extended periodic 1D-, 2D- or 3D-structures. The apparatus 100 comprises an atomic descriptors providing unit 110, a trained property model providing unit 120 and a property determination unit 130. Moreover, the apparatus can also comprise a property providing unit not shown in Fig. 1. In particular, the apparatus 100 can be realized in any form of software and/or hardware that can be dedicated to the task of determining properties of a substance or can provide additional further functions. The apparatus 100 can further comprise an input unit 141, like a keyboard, a mouse, a touchscreen etc., and/or an output unit 142 for outputting information to a user, for instance, on a display, as audio output, by utilizing a lighting unit, etc. However, the input unit 141 and the output unit 142 can also be omitted and the apparatus 100 can also be provided, for instance, as part of the software and/or hardware of a network of computing devices, for instance, a cloud, wherein in this case the apparatus can be connected to other computing devices that might act as input or output unit or that allow to provide information to the apparatus, for instance, in form of stored information and/or to receive information from the apparatus, for instance, a result of the determination, that can then be further processed without providing it explicitly as output to a user.

The atomic descriptors providing unit 110 is adapted to provide atomic descriptors, for instance, stored on a respective database of atomic descriptors or determined beforehand from respective knowledge of the specific molecule. Generally, the atomic descriptors are indicative of characteristics of atoms of the substance with respect to the structure of the substance. For example, in a preferred embodiment the atomic descriptors comprise atom specific quantities derived from a molecular electronic structure of the substance and/or atomic features indicative of the atomic structural environment of the atoms of the substance. The atomic descriptors providing unit 110 is then adapted to provide the atomic descriptors, for instance, to the property determination unit 130.

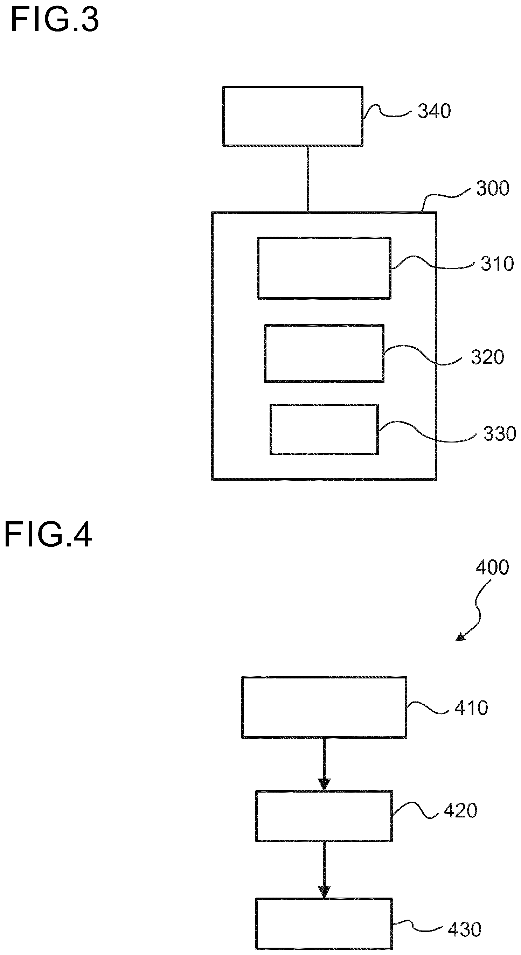

Further, the apparatus 100 comprises the trained property model providing unit 120 that is adapted to provide a trained machine learning based property model. The trained property model is adapted, i.e. has been trained, to determine a property of the substance as output when provided as input with the atomic descriptors. In particular, the properties of the substance that are determined by the trained property model can refer to physico-chemical and/or application properties. Physico-chemical properties refer to physical or chemical characteristics of a substance defined as being of either molecular nature or representing extended periodic 1D-, 2D- or 3D-structures, for instance, a melting point, a viscosity, etc. The application properties refer to application specific characteristics of the substance defined as being of either molecular nature or representing extended periodic 1D-, 2D- or 3D-structures, for instance, biodegradability, toxicity, flashpoint, etc. Generally, some properties might be considered as belonging to both physico-chemical properties and also application specific properties depending, for instance, on the respective intended application. Generally, the trained property model can be trained using, for instance, the training apparatus and the training method as will be described with respect to Figs. 3 and 4.

Generally, the trained property model comprises an atomic classifier adapted to classify atoms of the molecule into one or more atom classes based on the atomic descriptors and a regression model adapted to determine the property based on the atom classes determined for the molecule. In particular, the atomic classifier can refer to any machine learning classifier that can be trained to provide one or more classes for objects and to classify the object into the respective classes based on the provided characteristics of the objects, in this case, based on the provided atomic descriptors. The atom classes utilized by the atomic classifier are not predetermined, for instance, by user considerations or an expert class hierarchy, but are trained concurrently with the regression model such that for each respective case, for instance, for each respective property that should be determined, the optimal atom classes are identified by training alone. Thus, the atom classes of the atomic classifier are determined in a purely data-driven manner. Also, the regression model that utilizes the atom classes determined for the molecule to determine the property can refer to any machine learning based regression model. Preferably, the regression model refers to anyone of a linear regression, multilinear regression, an artificial neural network, Gaussian process regression, a Kernel algorithm, a support vector regression and a random forest algorithm.

In the following a preferred example for a classifier and the regression model is described in more detail. Preferably, the utilized classifier assigns to each atom of a substance a probability, e.g. in form of an atom class coefficient, of belonging to a certain atom group. This can be achieved during training by first applying a multi-linear regression to each atomic descriptor vector, yielding unnormalized atom class coefficients. This can then followed by a normalization via a softmax function. Atomic descriptor vectors can be used as input to the classifier either directly or after an additional transformation with e.g. an artificial neural network. Alternative normalization schemes to softmax can be employed, provided the optimization function of the property model is differentiable and the final atom class coefficients are non-negative and sum up to one for each atom. An example is taking the square of each value in the unnormalized atom class coefficient vectors and dividing by the sum of squared values. The normalized atom class assignments can then be used in the regression model to determine the final property, as well as to impose additional constraints on the training procedure. Examples include orthogonality constraints to encourage chemically different atoms to be assigned to different groups and entropy based constraints to limit the number of groups populated during training.

The trained property model providing unit 120 is then adapted to provide the trained property model, for instance, to the property determination unit 130. The property determination unit 130 can then be adapted to determine the property of the substance by applying the trained property model to the atomic descriptors, i.e. by providing the atomic descriptors of the substance as input to the trained property model. The output of the trained property model then refers or is indicative of the respective property for which the trained property model has been trained. Generally, property determination unit 130 can also be adapted to apply more than one property model to the atomic descriptors, for instance, to apply different property models trained for different respective properties such that the property determination unit 130 is adapted to determine more than one property of the substance. The different trained property models can then be applied subsequently or in parallel to the atomic descriptors. In particular, in some embodiments the property determination unit can be adapted to utilize one or more results, i.e. properties of the substance, determined by the trained property model again as atomic descriptors and/or to determine further atomic descriptors which can then again be provided to a respectively trained property model resulting in further properties of the substance.

Optionally the apparatus 10 can further comprise a property providing unit for providing the property for further processing. For example, the property providing unit can refer to the output unit 142 processing the property such that it can be provided to a user, for example, via a display. However, the property providing unit can also be configured as an interface for interfacing with other systems, for example, with a production process control system. In this case the further processing can refer to generating control data based on the determined property for controlling a production process control system. For instance, the production process can be controlled to produce the substance if the determined property meets a target property, or can be configured to set one or more process parameters of a production process based on the determined property, e.g. if the substance is utilized during the production and the respective determined property has an influence on the production parameters.

Fig. 2 shows schematically and exemplarily a method for determining properties of a substance. The method 200 comprises a step 210 of providing atomic descriptors as, for instance, described above. Moreover, the method 200 comprises a step 220 of providing a trained machine learning based property model. In particular, the trained machine learning based property model can refer to a property model, as described above, that has been trained, for instance, utilizing the apparatus and method as described with respect to Figs. 3 and 4. Generally, the step 210 of providing the atomic descriptors and the step 220 of providing a trained machine learning based property model can be performed in any arbitrary order or even at the same time. In a last step 230, the method 200 comprises then determining the property of the substance by applying the provided trained property model to the atomic descriptors.

Fig. 3 shows schematically and exemplarily a training apparatus 300 for training a machine learning based property model that can be utilized in the apparatus and method as described above. The training apparatus 300 comprises a training data providing unit 310, a trainable property model providing unit 320 and a training unit 330.

The training data providing unit 310 is adapted to provide training data for training the property model. In particular, the training data comprises atomic descriptors and correspondingly known properties of a plurality of different substances consisting of different substances, respectively. For example, the training data providing unit 310 can be connected to a database 340 storing a plurality of such data sets containing atomic descriptors of molecules and corresponding properties. Based on the desired training of the property model, for instance, based on the desired output of one or more respective properties, the training data providing unit 310 can also be adapted to select from the database 340 the respective training data for providing the same. However, data from such a database 340 can also be selected by a user or can be selected by the training data providing unit 310 based on specific criteria provided by a user.

The trainable property model providing unit 120 is then adapted to provide a trainable machine learning based property model. In particular, the structure of the trainable machine learning based property model can be in accordance with the structure of the trained property model as described above. Generally, a trainable property model can be regarded as referring to a property model for which the parameters determining the processing of input in the property model to generate the output of the property model are adaptable. For example, at the beginning, the parameters can be set to a predetermined value which is then adapted during the training of the property model based on the training data, such that the property model can later fulfil its function. However, the trainable property model can also refer to an already trained property model, for instance, to a property model that has already been trained with a respective training data set. In some cases it might be desirable to retrain such an already trained property model, for instance, for increasing an accuracy or for extending the applicability of the property model, for instance, to different substances or to further properties. In this case, the retraining of the property model can refer to providing a new training data set to the property model for further adapting the parameters of the trainable property model.

The training unit 330 is then adapted to train the provided property model based on the training data and the provided trainable property model. Preferably, the training of the property model refers to an iterative training in which the model parameters of the provided property models are optimized for their respective task based on the training data until a predetermined accuracy improvement is provided by the trained property model. In particular, a threshold can be set that allows to determine when the training of the property model has converged to a state in which, by an additional training, no further improvement beyond the respective threshold is to be expected. Moreover, it is preferred that the model parameters that can be divided into classifier parameters and regression parameters are trained concurrently, for instance, are optimized together with each iteration step.

Fig. 4 shows schematically and exemplarily a flow chart of a method for training a machine learning based property model. The method 400 comprises a step 410 of providing training data for training the property, for instance, in accordance with the above described principles. Further, the training method 400 comprises a step 420 of providing a trainable machine learning based property model. The trainable machine learning based property model has preferably a structure as already described above. Generally, the step 410 of providing training data and the step 420 of providing a trainable machine learning based property mode can be performed in any arbitrary order or even at the same time. Further, the method 400 comprises a step 430 of training the provided property model based on the training data such that the trained property model is adapted to determine a property of a substance consisting of a specific molecule as output when provided as an input with atomic descriptors of the specific molecule. Generally, the training can be performed according to any of the principles as already described above.

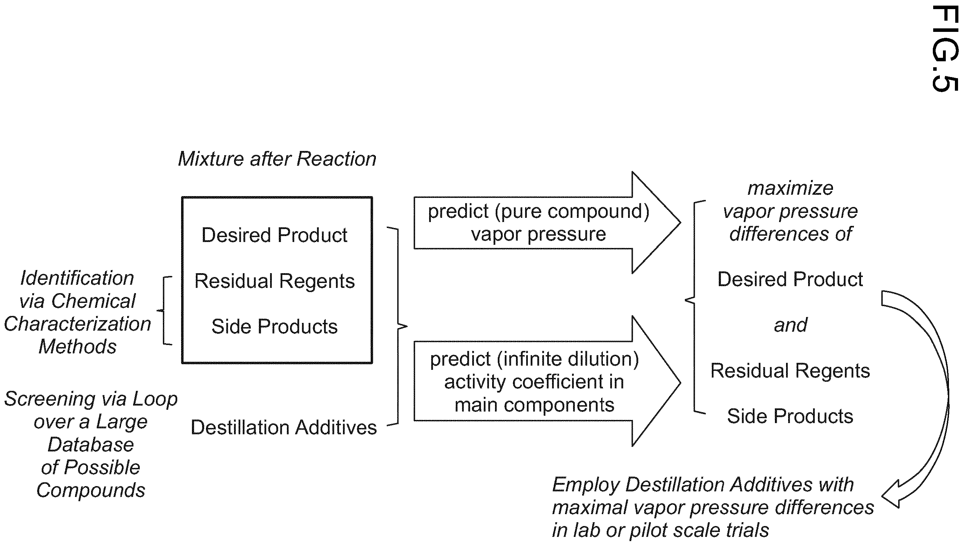

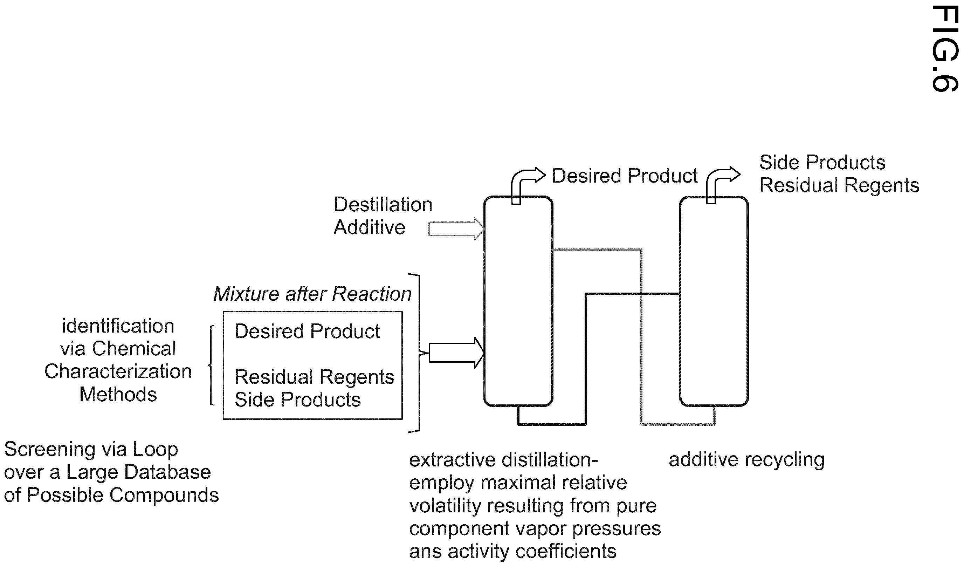

Fig. 5 and 6 show an exemplary application of the above described apparatus to reduce the energy effort in in the context of extractive distillation as a method to separate fluid mixtures. The quality of a chemical production process is often characterized by the purity of a product, which has to be achieved by the purity of the educts and the separation of side products that are created in the reaction process. If an ordinary distillation process is limited by a small separation factor of components, e.g. a product and similar side components, an additive can be provided for increasing the separation factor. A screening for such an additive using the above described apparatus can result in a technical and economical distillation process particularly with significant reduced energy effort, e.g. with an extractive distillation.

In order to screen for an optimal additive for an extractive distillation the following thermodynamic data are preferably utilized a) a pure component vapor pressure of the components, that have to be separated, and a pure component vapor pressure of the additive, and b) the activity coefficients or, as a first screening approximation, the activity coefficients at infinite dilution of the components, that have to be separated, with the additive as solvent. The selectivity can be defined as the ratio of the activity coefficients of the components to separate at infinite dilution in the additive solvent. The selectivity in combination with the pure component vapor pressure data is directly connected to the separation factor and the effort, i.e. distillation stages investment and energy consumption, of an extractive distillation applying an additive. Furthermore, the reciprocal values of the activity coefficients of the components in the additive can be applied as a first approximation for capacity criteria in order to avoid a two-phase splitting of the mixtures in the column.

The apparatus as described above can be utilized for determining the pure component vapor pressure and activity coefficients of different potential additives and thus allows to preselect potentially advantageous additives in relation to an enormous variety of components for an optimal distillation and separation process.

For example, the following equation can be utilized for optimizing with respect to a maximal relative volatility difference of a desired product with respect to other constituents, wherein respectively determined vapor pressures for the pure products and infinite dilution activity coefficients are utilized:

$$\text{relative volatility}(i \text{ vs } j) = \frac{\gamma_i \cdot p_i^0}{\gamma_j \cdot p_j^0} \approx \frac{\gamma_i^\infty \cdot p_i^0}{\gamma_j^\infty \cdot p_j^0}$$

In this equation $\gamma_i^\infty$ and $\gamma_j^\infty$ are the infinite dilution activity coefficients of the product and constituent, respectively, and $p°$ and $p_j^0$ are the determined vapor pressures for the respective pure products. The respective vapor pressures for the pure products and infinite dilution activity coefficients can be determined as property for the respective substances utilizing the apparatus and method as described above and an iterative optimization scheme can be used for determining a potential additive that maximized the relative volatility.

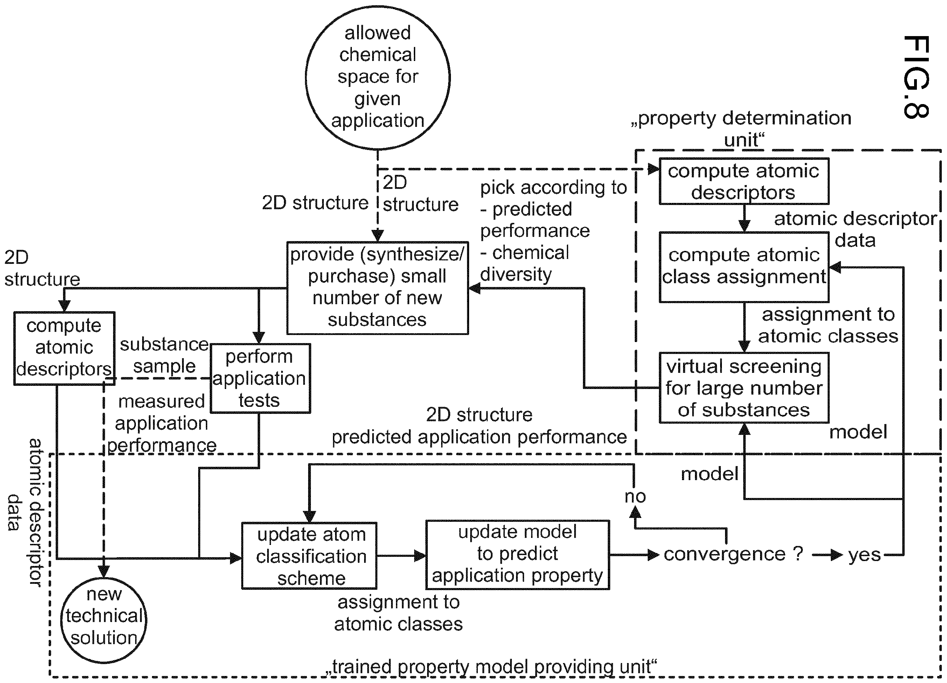

In a following further example, an application of the apparatus and method described above to the determination of temperature dependent pure substance vapor pressures is described, for instance, for being utilized for the application described above. The model consists in this application preferably of a SchNet model for determining suitable atomic descriptors, followed by a linear regression and softmax activation as classifier for classifying the atom groups, preferably, allowing a maximum of 50 different groups. The frequency of groups in a substance can then be used to determine the decadic logarithm of the vapor pressure via linear regression, followed by multiplication with a factor f dependent on the target temperature T and the reference normal boiling point TNBP of the substance. For example, the relation f=(T/TNBP - 1) / (T/TNBP - 0.125) can be utilized. The training data set can be determined based on vapor pressure curves and normal boiling points taken from the DIPPR dataset. In this example, the substances are constrained to contain only the elements C, H, N and O, yielding a set of 1016 substances. For each substance, the vapor pressure can be evaluated at 20 different temperatures within the limits specified in the DIPPR database, resulting in a total of 20580 data points. Of these, 80% can be used for training, 10% for validation and 10% for testing. The split can be performed in a random fashion with the condition that data belonging to the same compound can only occur in one of the subsets. The training procedure can utilize a Huber loss on the decadic logarithm of the determined and true pure substance vapor pressure. Structures optimized with density functional theory, as well as experimental temperatures and reference normal boiling points can serve as inputs to the regression model. The loss can be minimized using mini-batch stochastic gradient descent with the ADAM algorithm. Training can be monitored with an early stopping procedure operating on the validation set and stopping once the prediction error improvements stayed below a pre-defined threshold. The best model can then be chosen based on the lowest validation error achieved during training and its prediction error evaluated using the test set. The model generated by this procedure provides a prediction error of 21.25% on the pure substance vapor pressure of previously unseen substances, which is accurate enough for screening respective large sets of new substances for potentialtarget substances and can even be increased using the screening procedure described in Fig. 8.

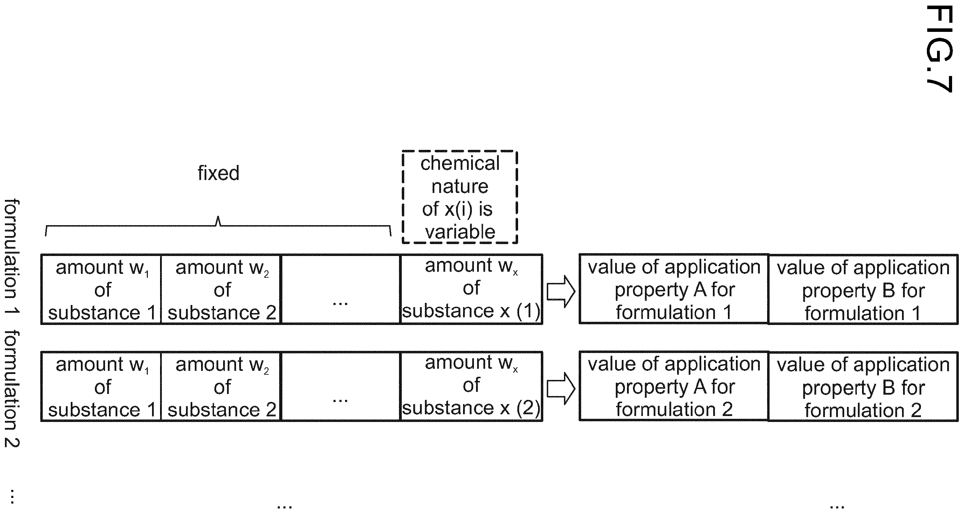

Fig. 7 shows schematically and exemplarily an application of the invention for optimizing a formulation. In this embodiment a target formulation should be provided that fulfils one or more predetermined target properties. The formulation comprises at least two, preferably, a plurality of components that can be any substance according to the present invention. In most cases one or more of the components of the target formulation are fixed, for instance, due to respective application requirements. However, in this example, at least one variable component is present within predetermined application limits. In particular, a list of a plurality of potential substances that can be used as additional component can be provided, optionally together with respective quantities of the potential substance as additional component. The apparatus and method as described above can then be utilized for determining one or more properties of a formulation comprising a first potential substance as additional component. In particular, the properties of the substances of the formulation can be determined and the respective relative amount of the substance can be utilized to determined a respective property for the formulation. The determined one or more properties can then be compared with the respective target properties, i.e. the respective values of the properties are compared with the respective value of the target property. Based on the comparison, it can then be determined whether the formulation meets the predetermined target, i.e. meets the one or more target properties, or not. If the formulation meets the target, the current formulation can be determined as target formulation. If the formulation does not meet the target a new formulation can be provided by changing the additional component and or the amount of the current component in accordance with the predetermined limits. In particular, respective rules can be utilized for automatically generating a new formulation based on the previous formulation. For example, the rule can determine that first an amount of the additional component is increased or decreased in predetermined increments until a respective predetermined limit is reached and then that the component is replaced by a new component of a list of potential components starting with a predetermined starting amount. However, also more sophisticated rules can be utilized.

Fig. 8 shows schematically and exemplarily a method for increasing an accuracy of the determination of a respective property model and/or for screening for new substances for an application. In this example, first a respective application a new substance and thus also for the property model, i.e. the determination of respective target properties, is provided. The intended application also determines the respective chemical space for the potential target substances. For example, in a human care application the chemical space must exclude potentially harmful substances. However, the chemical space can also be defined based on other considerations, like the availability of substances, the interaction with other components of a formulation, etc. Based on the application space a plurality of potential new substances can be determined that cover the application space. The above described apparatus and method can then be utilized for determining respective properties for these potential new substances very fast for screening the application space. In particular, for these potential new substances atomic descriptors are determined, the respectively trained classifier and regression algorithm is applied and the property determined accordingly. As a result the respective properties can be compared with the target property and potential new substances fulfilling the target property or meeting the target property within predetermined limits, which can be set broadly at the beginning, are selected. Additionally or alternatively some of the potential new substances in the application space can be selected based on other criteria, for instance, arbitrarily, or to cover the application space at least to some predetermined extend. The such select substances are then synthesized. This small number of substances can then be subject to respective application test or measurements of the atomic descriptors. For example, the application properties and/or atomic descriptors of these substances can be measured or derived from respective measurements. These measurement data can then be utilized for updating, in particular, retraining, the property model. The updated property model can then again be utilized for determining properties of new substances in the application space. This leads to a respective higher accuracy of the property model, in particular for regions of the application space, from which the measured substances have been selected. Thus, when applying the steps described above iteratively, for example, by screening in following steps only potential new substances in a region of the application space that are nearest to meeting the target property in the previous step, the property model becomes more and more accurate in regions in which a new target substance meeting the target property is expected.

In the following further examples and principles of the invention are described. Generally, the property model, for instance, as described above, is adapted to learn an assignment of atoms to different atom classes, based on training data comprising properties of a substance and atomic descriptors indicative of an atomic structural environment and/or atomic descriptors derived from electronic structure calculations. Suitable atomic classes and atomic descriptors for a specific property determination are determined in a purely data-driven fashion as part of an overall regression problem. The number and combination of atom classes present in a substance, which can be of molecular nature or an extended periodic material, are then used in a regression model for the determination of the properties.

In order to improve the generally high predictive power of this approach, high-quality training data and suitable structural and computational descriptors can be used. In this case the atom classes can be trained even more accurately with respect to the property of interest. Apart from models to directly determine a property, the invention as described above can also be applied to delta-learning, using a) a physical/engineering substance property determination model and b) the presented regression model to identify and correct systematic errors of the physical model.

Preferred embodiments for training the property model, for instance, using the above described training apparatus and method, will be described in the following. Preferably, the training data for setting up the property model comprises a) physico-chemical and/or application data for properties that depend on the chemical nature of respective substances, and b) atomic descriptors, i.e. features, derived from the atomic structural environment within these substances and/or obtained from electronic structure calculations of the respective substances.

Physico-chemical or application properties for which the property model can be trained can be either generally valid or depend on external conditions, e.g. temperature, pressure, the composition of a mixture, etc. For example, the properties for which the property model can be trained can refer to physico-chemical properties comprising one or more of are melting point, boiling point, vapor pressure, heat of vaporization, heat capacity, flashpoint and autoignition temperature, liquid density, critical temperature and pressure, electric and thermal conductivity, glass transition temperature, viscosity, surface tension, refractive index, mechanical properties, total energy, dipole moment, polarizability, HOMO-LUMO- or bandgap, ionization potential, electron affinity, activity coefficient, partition coefficient, solubility, e.g. in all types of equilibria between solids, liquids and gases, cloud point, critical micelle concentration, acid- and base dissociation constants, hydrolytic stability, corrosivity, catalytic activity for a given chemical reaction or spectroscopic properties, e.g. transition energies and intensities in e.g. IR- and UV-Vis-spectra,. Moreover, the properties can refer to application properties referring to a) general properties beyond pure physical chemistry, e.g. the various types of toxic and ecotoxic behavior, biodegradability in various habitats, odor, ozone depletion potential, global warming potential, and/or b) action as an additive in mixtures, e.g. influence on octane and cetane number of fuels, stabilization against UV-radiation or oxidation, flame retardancy, influence on haptics.

The derivation of atomic descriptors from an atomic structural environment can be performed according to any formalism which can transform a three-dimensional structure and elemental composition of a molecule system to a set of atom-wise descriptors. For example, end-to-end architectures, e.g. SchNet, PhysNet, can be also trainable and can learn to determine a suitable set of atomic descriptors directly from the structural data and can hence adapt to a wide range of different chemical systems, provided that sufficient data is available. Alternatively, pre-defined atomic descriptors, e.g. ANI, SOAP, FCHL, can be used, which will offer increased accuracy for small data sets. There is the possibility that multiple conformers of a molecule are relevant for a property. In order to take this into account, atomic descriptors can be derived from multiple conformers and combined using different schemes, for example, weighted averaging, where the weights can either be the Boltzmann weights of the conformers or learned during the training procedure of the property model.

Atomic descriptors referring to atom-specific quantities can be derived from molecular electronic structure calculations and can refer, for instance, to one or more of partial charge, exposed surface fraction, averaged surface screening charge density from continuum solvation model calculations, averaged atomic radius in continuum solvation model calculations based on using a flexible, e.g. iso-density cavity, contributions to energy or free energy in COSMO-RS- or COSMO-SAC-like solvation models, e.g. misfit-, H-bond-donating, H-bond-accepting, dispersive or total residual contribution, pairwise contact probabilities from COSMO-RS- or COSMO-SAC-like solvation models, NMR-shielding constant. Also for these atomic descriptors the same way to account for conformational isomerism as above can be applied.

The trainable property model is then adapted to be trained, based on the atomic descriptors of molecules of the training data, for example, as described above, to assign the atoms of the molecule to different atom classes, which are then used to train the regression as provided by the regression model. Generally, apart from a simple multilinear regression also more complex strategies like artificial neural networks (ANNs), kernel methods, support vector regression or random forests can be utilized by the regression model. During the training the overall regression problem provided by the training data applied to the property model can be optimized, preferably, in an iterative way, during which optimal parameters/coefficients and atom classes are determined concurrently for the property model. Also the choice of actually used atomic descriptors can be optimized during the training.

The property model can be adapted to perform the atom class assignment via a classification layer such as softmax. This step offers quite some flexibility, for instance, the classifier can be adapted to introduce orthogonality during the optimization. Moreover, the classifier can be restricted to choose atom classes element specific, i.e., an N atom and an O atom will never share the same atom class. Without this restriction the classifier is more flexible, has the advantage of working with less atom classes altogether and can also improve transferability, i.e. to obtain models that work for elements for which only a few or even no data were available in the training set. Furthermore, the classifier can be adapted such that an atom can be either fully attributed to one atom class or also have several assignments. In the latter case, the importance of the different assignments can be mapped by atomic class weights. For example, the sum of weights can then be enforced to sum up to one, but this is not necessary. Generally, the higher flexibility in the latter case can be used to describe the differently strong potentially non-linear effect of different atoms on a given property.

As mentioned before, for the regression model determining the target property, either a simple multilinear regression can be employed or more complex strategies like artificial neural networks (ANNs), kernel methods, support vector regression or random forests can be utilized. The training procedure of the property model as performed, for instance, by the training unit of the training apparatus, yields both assigned atomic classes as well as determined values for the target property. Generally, the iterative training is finished if the prediction error and optional atom-class constraints, e.g. orthogonality, are converged within a given threshold.

A property model trained according to the procedure described above is able to determine the substance target property based either on the atomic descriptors, wherein the atomic descriptors can be derived from a structure of the molecule. This can be structures obtained from experimental techniques, e.g. X-ray diffraction, but the probably more relevant use case for the presented model is the derivation of molecular properties from computed structures and descriptors. This means, only computations and no experiments are needed to determine the target property, which is a huge advantage e.g. if a) a virtual screening is performed, where the candidates of interest are difficult to purchase or even have to be synthesized or if b) for a compound, although being easily available, the property of interest involves an expensive, error-prone or hazardous measurement procedure.

In order to perform such a determination based on molecular or periodic computations, the first step represents one or several calculation(s) to obtain the atomic descriptors referring for instance to the structure(s) of one, typically, at the application conditions, the most stable, conformer or of an ensemble of relevant conformers. In case of a conformer ensemble, also conformational weights according to the Boltzmann-equation have to be computed from relative energies or free energies, if available. Depending on the trained atomic descriptors the atomic descriptors can then be determined, for instance from the respective structure. With this, all input parameters are available for determining the target property with the above described property model.

The determined physico-chemical or application properties can generally be used to speed-up product or process development by drastically reducing the time until there is information available how a new compound would behave or how a compound would behave at different conditions. In the specific case, that as target properties also total energies of substances are learned, the obtained total energies plus their gradients with respect to nuclear coordinates based on atom classes can also serve as the computational method for determining most probable molecular or periodic structures as well as their ensembles as mentioned above.

The general benefits of the availability of predictive models for physico-chemical or application properties based only on computations have already been given in the above section. Further, specific advantages of the presented invention are, for example, that no elaborate time-consuming, model-developer-dependent and potentially biased hierarchical assignment of groups has to be provided. Moreover, the atom classes can be assigned for each property in a different way, which reflects that for different properties also different atoms can play a dominant role. The invention is generally, also applicable to molecules with unclear/ambiguous chemical binding situations, e.g. inorganic substances, metallo-organic compounds, molecules with non-covalent interactions, where the molecular graph derived by chemoinformatic tools is sensitive to small changes in bond lengths, and the assignment to hierarchical functional groups is thus error-prone. Apart from directly determining a property, the invention is particularly useful also for delta-learning, using a) a physical determination model and b) the presented atom class based model to identify and correct systematic errors of physical model. Moreover, insight into atomic and thus also functional group similarity at an unbiased level can be provided by the property model, opening up new ways to the interpretation of how the properties actually materialize and thus inspiring ideas how to improve them.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the providing of the atomic descriptors and properties, the providing of the property model, the applying or training of the property model, etc., performed by one or several units or devices can be performed by any other number of units or devices. These procedures can be implemented as program code means of a computer program and/or as dedicated hardware.

A computer program product may be stored/distributed on a suitable medium, such as an optical storage medium or a solid-state medium, supplied together with or as part of other hardware, but may also be distributed in other forms, such as via the Internet or other wired or wireless telecommunication systems.

Any units described herein may be processing units that are part of a classical computing system. Processing units may include a general-purpose processor and may also include a field programmable gate array (FPGA), an application specific integrated circuit (ASIC), or any other specialized circuit. Any memory may be a physical system memory, which may be volatile, non-volatile, or some combination of the two. The term "memory" may include any computer-readable storage media such as a non-volatile mass storage. If the computing system is distributed, the processing and/or memory capability may be distributed as well. The computing system may include multiple structures as "executable components". The term "executable component" is a structure well understood in the field of computing as being a structure that can be software, hardware, or a combination thereof. For instance, when implemented in software, one of ordinary skill in the art would understand that the structure of an executable component may include software objects, routines, methods, and so forth, that may be executed on the computing system. This may include both an executable component in the heap of a computing system, or on computer-readable storage media. The structure of the executable component may exist on a computer-readable medium such that, when interpreted by one or more processors of a computing system, e.g., by a processor thread, the computing system is caused to perform a function. Such structure may be computer readable directly by the processors, for instance, as is the case if the executable component were binary, or it may be structured to be interpretable and/or compiled, for instance, whether in a single stage or in multiple stages, so as to generate such binary that is directly interpretable by the processors. In other instances, structures may be hard coded or hard wired logic gates, that are implemented exclusively or near-exclusively in hardware, such as within a field programmable gate array (FPGA), an application specific integrated circuit (ASIC), or any other specialized circuit. Accordingly, the term "executable component" is a term for a structure that is well understood by those of ordinary skill in the art of computing, whether implemented in software, hardware, or a combination. Any embodiments herein are described with reference to acts that are performed by one or more processing units of the computing system. If such acts are implemented in software, one or more processors direct the operation of the computing system in response to having executed computer-executable instructions that constitute an executable component. Computing system may also contain communication channels that allow the computing system to communicate with other computing systems over, for example, network. A "network" is defined as one or more data links that enable the transport of electronic data between computing systems and/or modules and/or other electronic devices. When information is transferred or provided over a network or another communications connection, for example, either hardwired, wireless, or a combination of hardwired or wireless, to a computing system, the computing system properly views the connection as a transmission medium. Transmission media can include a network and/or data links which can be used to carry desired program code means in the form of computer-executable instructions or data structures and which can be accessed by a general-purpose or special-purpose computing system or combinations. While not all computing systems require a user interface, in some embodiments, the computing system includes a user interface system for use in interfacing with a user. User interfaces act as input or output mechanism to users for instance via displays.

Those skilled in the art will appreciate that at least parts of the invention may be practiced in network computing environments with many types of computing system configurations, including, personal computers, desktop computers, laptop computers, message processors, hand-held devices, multi-processor systems, microprocessor-based or programmable consumer electronics, network PCs, minicomputers, mainframe computers, mobile telephones, PDAs, pagers, routers, switches, datacenters, wearables, such as glasses, and the like. The invention may also be practiced in distributed system environments where local and remote computing system, which are linked, for example, either by hardwired data links, wireless data links, or by a combination of hardwired and wireless data links, through a network, both perform tasks. In a distributed system environment, program modules may be located in both local and remote memory storage devices.

Those skilled in the art will also appreciate that at least parts of the invention may be practiced in a cloud computing environment. Cloud computing environments may be distributed, although this is not required. When distributed, cloud computing environments may be distributed internationally within an organization and/or have components possessed across multiple organizations. In this description and the following claims, "cloud computing" is defined as a model for enabling on-demand network access to a shared pool of configurable computing resources, e.g., networks, servers, storage, applications, and services. The definition of "cloud computing" is not limited to any of the other numerous advantages that can be obtained from such a model when deployed. The computing systems of the figures include various components or functional blocks that may implement the various embodiments disclosed herein as explained. The various components or functional blocks may be implemented on a local computing system or may be implemented on a distributed computing system that includes elements resident in the cloud or that implement aspects of cloud computing. The various components or functional blocks may be implemented as software, hardware, or a combination of software and hardware. The computing systems shown in the figures may include more or less than the components illustrated in the figures and some of the components may be combined as circumstances warrant.

Any reference signs in the claims should not be construed as limiting the scope.

The invention refers to an apparatus for determining properties of a substance. A providing unit provides atomic descriptors that are indicative of characteristics of atoms of the substance with respect to the structure of the specific molecule. A model providing unit provides a trained property model, wherein the trained property model has been trained to determine a property of the substance as output when provided with the atomic descriptors.

The property model comprises a) an atomic classifier adapted to classify atoms of the substance into one or more atom classes based on the atomic descriptors and b) a regression model adapted to determine the property based on the atom classes determined for the substance. A determination unit determines the property of the substance by applying the trained property model to the atomic descriptors.

## Claims

1. An apparatus for determining properties of a substance defined as being of either molecular nature or representing extended periodic 1D-, 2D- or 3D-structures, wherein the apparatus (100) comprises:

   an atomic descriptors providing unit (110) for providing atomic descriptors that are indicative of characteristics of atoms of the substance with respect to the structure of the substance,
   a trained property model providing unit (120) for providing a trained machine learning based property model, wherein the trained property model is adapted to determine a property of the substance as output when provided as input with the atomic descriptors, wherein the property model comprises a) an atomic classifier adapted to classify atoms of the substance into one or more atom classes based on the atomic descriptors and b) a regression model adapted to determine the property based on the atom classes determined for the substance,
   a property determination unit (130) for determining the property of the substance by applying the trained property model to the atomic descriptors, and
   a property providing unit for providing the property for further processing.

**2.** The apparatus according to claim 1, wherein the atomic descriptors comprise atom specific quantities derived from an electronic structure of the substance and/or atomic features indicative of the atomic structural environment of the atoms of the substance.

**3.** The apparatus according to any of claims 1 and 2, wherein the properties of the specific molecule comprise physicochemical and/or application properties, wherein physicochemical properties refer to physical or chemical characteristics of a substance consisting of the specific molecule, and wherein application properties refer to application specific characteristics of a substance.

**4.** The apparatus according to any of the preceding claims, wherein, if the substance is of a molecular nature and comprises molecules in different conformations, the atomic descriptors of the substance refer to a weighted average of the atomic descriptors of the different conformers.

**5.** The apparatus according to claim 4, wherein the weights of the weighted averaging refer to Boltzmann weights or refer to trained weights trained together with the property model based on the same training data used for training the property model.

**6.** The apparatus according to any of the preceding claims, wherein the atomic classifier is adapted to classify the atoms of the substance into the one or more atom classes such that atoms referring to different elements are classified into different atom classes.

**7.** The apparatus according to any of the preceding claims, wherein the atomic classifier is adapted to classify an atom of the substance into one or more atom classes, wherein, if the atom is classified into more than one atom class, an atomic class weight is provided to each atom class to which the atom has been assigned based on the probability that the atom belongs the respective atom class.

**8.** The apparatus according to any of the preceding claims, wherein the regression model refers to anyone of a multilinear regression, an artificial neural network, a Kernel algorithm, a support vector regression and a random forest algorithm.

**9.** A training apparatus for training a machine learning based property model, wherein the apparatus (300) comprises:

a training data providing unit (310) for providing training data for training the property model, wherein the training data comprises atomic descriptors and a corresponding known property of a plurality of different substances, respectively,

a trainable property model providing unit (320) for providing a machine learning based property model, wherein the property model comprises a) an atomic classifier trainable to classify atoms of a substance into one or more atom classes based on atomic descriptors, and b) a regression model trainable to determine the property based on the atom classes determined for the molecule, and

a training unit (330) for training the provided property model based on the training data such that the trained property model is adapted to determine a property of a substance as output when provided as an input with atomic descriptors of the substance.

**10.** A system for screening for a potential substance in a predefined application, wherein the system comprises:

an apparatus according to claim 1,

a target property providing unit for providing a target property of a target substance and a potential target substance, and

a screening unit, wherein the screening unit is adapted to utilize the apparatus to determine a respective property of the potential target substances and to compare the determined property of the potential target substance with the target property and, based on the comparison, either i) determining the potential target substance as the target substance, or ii) providing a new potential target substance and repeating the determination of the property utilizing the new potential target substance.

**11.** A method for determining properties of a substance defined as being of either molecular nature or representing extended periodic 1D-, 2D- or 3D-structures, wherein the method (200) comprises:

providing (210) atomic descriptors that are indicative of characteristics of atoms of the substance with respect to the structure of the substance,

providing (220) a trained machine learning based property model, wherein the trained property model is adapted to determine a property of the substance as output when provided as input with the atomic descriptors, wherein the property model comprises a) an atomic classifier adapted to classify atoms of the substance into one or more atom classes based on the atomic descriptors and b) a regression model adapted to determine the property based on the atom classes determined for the substance,

determining (230) the property of the substance by applying the trained property model to the atomic descriptors, and
providing the property for further processing.

12. A method for screening for a potential substance in a predefined application, wherein the method comprises:

providing a target property of a target substance and a potential target substance, and
utilizing a method according to claim 11 to determine a respective property of the potential target substances and to compare the determined property of the potential target substance with the target property and, based on the comparison, either i) determining the potential target substance as the target substance, or ii) providing a new potential target substance and repeating the determination of the property utilizing the new potential target substance.

13. A training method for training a machine learning based property model, wherein the method (400) comprises:

providing (410) training data for training the property model, wherein the training data comprises atomic descriptors and a corresponding known property of a plurality of different substances, respectively,
providing (420) a machine learning based property model, wherein the property model comprises a) an atomic classifier trainable to classify atoms of a substance into one or more atom classes based on atomic descriptors, and b) a regression model trainable to determine the property based on the atom classes determined for the substance, and
training (430) the provided property model based on the training data such that the trained property model is adapted to determine a property of a substance as output when provided as an input with atomic descriptors of the substance.

14. A computer program product for training a machine leaning based property model, wherein the computer program product comprises program code means for causing the property model training apparatus of claim 9 to execute the training method according to claim 13.

15. A computer program product for determining properties of a substance, wherein the computer program product comprises program code means for causing the apparatus of any of claims 1 to 8 to execute the method according to claim 11.

# FIG.1

# FIG.2

# FIG.3

# FIG.4

FIG.5

# FIG.6

Side Products
Residual Regents

Desired Product

Destillation
Additive

*Mixture after Reaction*

Desired Product

Residual Regents
Side Products

identification
via Chemical
Characterization
Methods

Screening via Loop
over a Large Database
of Possible Compounds

additive recycling

extractive distillation-
employ maximal relative
volatility resulting from pure
component vapor pressures
ans activity coefficients

# FIG.7

**formulation 1**

| amount $w_1$ of substance 1 | amount $w_2$ of substance 2 | ... | amount $w_x$ of substance x (1) | ⇨ | value of application property A for formulation 1 | value of application property B for formulation 1 |
|---|---|---|---|---|---|---|

**formulation 2**

| amount $w_1$ of substance 1 | amount $w_2$ of substance 2 | ... | amount $w_x$ of substance x (2) | ⇨ | value of application property A for formulation 2 | value of application property B for formulation 2 |
|---|---|---|---|---|---|---|

...

fixed

chemical nature of $x(i)$ is variable

formulation 1   formulation 2   ...

FIG.8

"property determination unit"

compute atomic descriptors → atomic descriptor data → compute atomic class assignment → assignment to atomic classes → virtual screening for large number of substances → model

model

pick according to
- predicted performance
- chemical diversity

allowed chemical space for given application

2D structure

2D structure

provide (synthesize/purchase) small number of new substances

perform application tests

substance sample

measured application performance

2D structure
predicted application performance

no

convergence ? → yes

update model to predict application property

assignment to atomic classes

update atom classification scheme

compute atomic descriptors

2D structure

atomic descriptor data

new technical solution

"trained property model providing unit"

EP 4 386 765 A1

**EUROPEAN SEARCH REPORT**

Application Number

EP 22 21 4055

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 102 930 181 A (UNIV SICHUAN) 13 February 2013 (2013-02-13) * the whole document * | 1-15 | INV. G16C20/30 G06N3/00 |
| X | ZHANG Q. ET AL: "A QSPR approach for the fast estimation of DFT/NBO partial atomic charges", CHEMOMETRICS AND INTELLIGENT LABORATORY SYSTEMS, ELSEVIER SCIENCE PUBLISHERS B.V. AMSTERDAM, NL, vol. 134, 25 March 2014 (2014-03-25), pages 158-163, XP028659197, ISSN: 0169-7439, DOI: 10.1016/J.CHEMOLAB.2014.03.011 * the whole document * | 1-15 | ADD. G16C20/70 |
| X | CN 115 274 008 A (SUZHOU CHUANGTENG SOFTWARE CO LTD) 1 November 2022 (2022-11-01) * the whole document * | 1-15 | |
| X | RODRÍGUEZ-PÉREZ R. ET AL: "Support Vector Machine Classification and Regression Prioritize Different Structural Features for Binary Compound Activity and Potency Value Prediction", ACS OMEGA, vol. 2, no. 10, 4 October 2017 (2017-10-04), pages 6371-6379, XP093048801, US ISSN: 2470-1343, DOI: 10.1021/acsomega.7b01079 Retrieved from the Internet: URL:http://pubs.acs.org/doi/pdf/10.1021/ac somega.7b01079> * the whole document * | 1-15 | |

TECHNICAL FIELDS SEARCHED (IPC)

G16C
G06N

-/--

The present search report has been drawn up for all claims

| Place of search | Date of completion of the search | Examiner |
|---|---|---|
| The Hague | 26 May 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**
**European Patent Office**
**Office européen des brevets**

# EUROPEAN SEARCH REPORT

Application Number

EP 22 21 4055

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RAFIDHA RAHIMAN K. A. ET AL: "Using Neural Network classifier Support Vector Machine Regression for the prediction of Melting Point of Drug – like compounds", EMERGING TRENDS IN ELECTRICAL AND COMPUTER TECHNOLOGY (ICETECT), 2011 INTERNATIONAL CONFERENCE ON, IEEE, 23 March 2011 (2011-03-23), pages 636-640, XP031862981, DOI: 10.1109/ICETECT.2011.5760195 ISBN: 978-1-4244-7923-8 * the whole document * | 1-15 | |
| A | CN 114 496 304 A (UNIV SHANDONG) 13 May 2022 (2022-05-13) * the whole document * | 1-15 | |
| A | RODRÍGUEZ-PÉREZ R. ET AL: "Evolution of Support Vector Machine and Regression Modeling in Chemoinformatics and Drug Discovery", JOURNAL OF COMPUTER-AIDED MOLECULAR DESIGN, SPRINGER NETHERLANDS, NL, vol. 36, no. 5, 19 March 2022 (2022-03-19), pages 355-362, XP037920966, ISSN: 0920-654X, DOI: 10.1007/S10822-022-00442-9 [retrieved on 2022-03-19] * the whole document * | 1-15 | TECHNICAL FIELDS SEARCHED (IPC) |

The present search report has been drawn up for all claims

| Place of search | Date of completion of the search | Examiner |
|---|---|---|
| The Hague | 26 May 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 4055

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-2023

| Patent document cited in search report | | Publication date | Patent family member(s) | Publication date |
|---|---|---|---|---|
| CN 102930181 | A | 13-02-2013 | NONE | |
| CN 115274008 | A | 01-11-2022 | NONE | |
| CN 114496304 | A | 13-05-2022 | NONE | |

EPO FORM P0459

For more details about this annex : see Official Journal of the European Patent Office, No. 12/82